# EUROPEAN PATENT APPLICATION

(11) **EP 4 834 926 A1**
(43) Date of publication of application: **04.11.2026**
(21) Application number: 24911424.0
(22) Date of filing: 27.12.2024
(51) Int. Cl.: C07K 14/725, C07K 19/00, C12N 15/12, C12N 15/62, C12N 15/867, C12N 5/10, A61K 38/17, A61K 35/17, A61P 35/00

(54) **T CELL RECEPTOR TARGETING POLYPEPTIDE WITH KRAS G12V MUTATION AND USE OF T CELL RECEPTOR**

(30) Priority: 29.12.2023 CN 202311870153
(71) Applicant: Immuxell Biotech Ltd., Shanghai 201315 (CN)
(72) Inventor: HU, Hong-Ming, Shanghai 201315 (CN); YU, Guangjie, Shanghai 201315 (CN)
(74) Representative: Vossius & Partner Patentanwälte Rechtsanwälte mbB
(86) International application number: PCT/CN2024/143046
(87) International publication number: WO 2025/140504

(57) **Abstract**

Provided in the present invention are a T cell receptor targeting a polypeptide with a KRAS G12V mutation and the use of the T cell receptor. The T cell receptor molecule of the present invention specifically targets the KRAS G12V mutation, and the CDR3 sequence of the α chain variable region of the T cell receptor molecule contains CAVNPNTGNQFYF (SEQ ID NO: 1) or a mutant of SEQ ID NO: 1, and/or the CDR3 sequence of the β chain variable region contains CASSQDYGPQETQYF (SEQ ID NO: 2) or a mutant of SEQ ID NO: 2. Further provided in the present invention are a nucleic acid molecule encoding the T cell receptor molecule, a multivalent complex and dual-targeting protein molecule containing the T cell receptor molecule, a nucleic acid construct, a cell expressing the T cell receptor molecule, etc.

## Description

### TECHNICAL FIELD

The present invention relates to a T cell receptor targeting a polypeptide with the KRAS G12V mutation and nucleotide sequences encoding the same. The present invention also relates to a TCR-T cell therapy method developed based on the T cell receptor, as well as a dual-targeting anti-tumor protein drug developed based on the T cell receptor.

### BACKGROUND

Adoptive cellular immunotherapy is a newly developed cutting-edge technology that has achieved unprecedented success in the treatment of hematological malignancies. However, adoptive cellular immunotherapy technology faces many difficulties in the treatment of solid tumors. Finding suitable targets and developing receptor molecules that specifically bind to these targets could open up new prospects for solid tumor therapy. The KRAS gene has come into the researchers' view. KRAS is one of the main members of the Ras gene family (including NRAS, HRAS, and KRAS). It is a murine sarcoma virus oncogene located on chromosome 12, approximately 35 kb in length, encoding the KRAS protein. When KRAS mutates, it continuously binds to GTP, exhibits tyrosine kinase activity, and activates downstream signaling pathways, thereby causing uncontrolled cell proliferation and leading to tumorigenesis. Studies have found that KRAS mutations are present in approximately 30% of tumors, including 90% of pancreatic cancers, 50% of colon cancers, and 25% of lung cancers.

KRAS mutations often occur at positions such as glycine at position 12, glycine at position 13, and glutamine at position 61. Mutations at glycine position 12 and glycine position 13 account for as much as 97%, mainly including G12C, G12D, G12V, G12R, and G13D mutations. KRAS G12V mutation is expressed in approximately 30% of pancreatic cancers and 10% of colorectal cancer or non-small cell lung cancer patients. Moreover, RAS family members also share the G12V hotspot mutation in different cancer types (e.g., NRAS in melanoma). Although KRAS mutations are found in many tumors, KRAS has been regarded as an undruggable target for many years due to the lack of a pocket on its surface for binding small molecule inhibitors. Currently, there is only one KRAS G12C inhibitor, AMG 510, approved for marketing for the treatment of patients with advanced non-small cell lung cancer (NSCLC) carrying the KRAS G12C mutation who have received prior systemic therapy. However, KRAS G12C is rarely found in other cancers (such as pancreatic cancer, colon cancer, etc.). For other high-abundance KRAS mutation types, such as G12D and G12V, new therapeutic methods are also urgently needed.

Targeting high-abundance KRAS mutations with adoptive cellular immunotherapy is currently a breakthrough point in research in this field. One type of adoptive cellular immunotherapy, referred to as T cell receptor-engineered T cell therapy (TCR-T), has exhibits significant advantages. TCR-T therapy utilizes the tumor-killing properties of T cells, transfects tumor-specific TCR genes into T cells, and mediates the specific recognition of tumor antigens by T cells through the expressed receptors, ultimately achieving tumor recognition and killing effects. TCR-T cell therapy has demonstrated good safety and efficacy in clinical trials worldwide for the treatment of refractory relapsed melanoma, synovial sarcoma, multiple myeloma, and lung cancer, among others. Finding TCR receptors with high specificity and strong affinity is a key part and technical obstacle of TCR-T technology.

### SUMMARY

A first aspect of the present invention provides a T cell receptor (TCR) molecule, wherein the TCR molecule specifically targets the KRAS G12V mutation, a CDR3 sequence of an α chain variable region thereof comprises CAVNPNTGNQFYF (SEQ ID NO: 1) or a mutant of SEQ ID NO: 1, and/or a CDR3 sequence of a β chain variable region thereof comprises CASSQDYGPQETQYF (SEQ ID NO: 2) or a mutant of SEQ ID NO: 2.

A second aspect of the present invention provides a multivalent TCR complex, wherein the multivalent TCR complex comprises two or more TCR molecules according to any embodiment of the present invention.

A third aspect of the present invention provides a dual-targeting protein molecule capable of simultaneously binding to a tumor cell and an immune cell, wherein the dual-targeting protein molecule comprises the TCR molecule targeting the KRAS G12V mutation on the surface of a tumor cell according to any embodiment herein and a single-chain antibody (scFv) for recruiting and redirecting an immune cell to the periphery of the tumor cell, wherein a signal peptide and a transmembrane domain in the α chain variable region and the β chain variable region of the TCR molecule are deleted.

A fourth aspect of the present invention provides a nucleic acid molecule, wherein the nucleic acid molecule comprises a nucleic acid sequence encoding the TCR molecule or the dual-targeting protein molecule according to any embodiment of the present invention, or a complementary sequence thereof.

A fifth aspect of the present invention provides a nucleic acid construct, wherein the nucleic acid construct comprises the nucleic acid molecule according to any embodiment of the present invention.

A sixth aspect of the present invention provides an isolated cell, wherein the cell:
(1) comprises the nucleic acid construct according to any embodiment of the present invention or has the nucleic acid molecule according to any embodiment of the present invention integrated into its chromosome, and/or
(2) expresses the TCR molecule according to any embodiment of the present invention or the dual-targeting protein molecule according to any embodiment of the present invention.

Preferably, the cell is an immune effector cell, preferably a T cell, an NK cell, or a TIL cell.

A seventh aspect of the present invention provides a pharmaceutical composition, wherein the pharmaceutical composition comprises a pharmaceutically acceptable carrier and the TCR molecule, TCR complex, dual-targeting protein molecule, nucleic acid molecule, recombinant expression vector, or cell according to any embodiment of the present invention.

An eighth aspect of the present invention provides use of the TCR molecule, TCR complex, dual-targeting protein molecule, nucleic acid molecule, recombinant expression vector, or cell according to any embodiment of the present invention in the manufacture of a medicament for treating or preventing a disease related to a KRAS G12V mutant antigen in a patient.

A ninth aspect of the present invention provides a method for treating and/or preventing a disease related to a KRAS G12V mutant antigen in a patient, wherein the method:
comprising the step of adoptively transferring to the patient a T cell comprising the vector of the present invention or having the nucleic acid molecule of the present invention integrated into its chromosome, and/or expressing the TCR molecule according to any embodiment herein in the patient, or
comprising the step of administering to the patient the dual-targeting protein molecule according to any embodiment of the present invention or a pharmaceutical composition comprising the dual-targeting protein molecule.

The above aspects of the invention are described in detail as follows.

### BRIEF DESCRIPTION OF THE DRAWINGS

Figure 1: Schematic diagram of the pMSGV1_02-1 TCR vector.
Figure 2: Results of the preparation of TCR-J reporter cells and the detection of in vitro antigenic peptide activation in Example 4.
Figure 3: Results of the binding assay between the 02-1 TCR and peptide-MHC complexes in Example 6.
Figure 4: Results of the specificity assay of KRAS mutant antigen peptide activation of 02-1 TCR-T cells in Example 7.
Figure 5: Results of the sensitivity assay of KRAS mutant antigen peptide activation of 02-1 TCR-T cells in Example 7.
Figure 6: Results of the activation effect of KRAS mutant tumor cells on 02-1 TCR-T cells in Example 8.
Figure 7: Results of the cytotoxic effect of 02-1 TCR-T cells on KRAS mutant tumor cells in Example 9.

### DETAILED DESCRIPTION

The present invention has discovered a TCR molecule that specifically targets a KRAS G12V mutant antigen (particularly specifically targets the KRAS G12V mutant antigen set forth in SEQ ID NO: 16). The TCR molecule is capable of specifically binding to the KRAS G12V/HLA-A*11:01 complex on the surface of tumor cells, while normal non-cancerous cells are not recognized as they express an unmutated wild-type KRAS protein. Therefore, the TCR molecule of the present invention has strong specificity, which reduces the toxic side effects when T cells expressing the TCR molecule are used as a medicament, and does not cause damage to normal non-cancerous cells. The TCR molecule has broad applicability in the treatment of tumors (e.g., pancreatic cancer, colorectal cancer, lung cancer, endometrial cancer, ovarian cancer, prostate cancer, etc., particularly pancreatic cancer), thereby completing the present invention.

The present invention will be described in detail below. It should be understood that, within the scope of the present invention, the above-described technical features of the present invention and the technical features specifically described below (e.g., in the Examples) can be combined with each other to form preferred technical solutions.

### Definition of Terms

As used herein, TCR has a meaning well-known in the art. It is a glycoprotein on the cell membrane surface existing in the form of an α/β chain or γ/δ chain heterodimer, and is a characteristic surface marker of all T cells. The T cell receptor forms a T cell receptor complex together with the constant CD3 molecule. TCR is a receptor for intracellular antigenic peptides presented by the major histocompatibility complex (MHC). The TCR of most T cells is a dimer composed of α and β peptide chains, while the TCR of a minority of T cells is composed of γ and δ peptide chains. Each subunit contains two extracellular domains: a variable region and a constant region. The constant region is close to the cell membrane and linked to a transmembrane region, while the variable region is responsible for recognizing the peptide/MHC complex. Each variable region contains three highly variable complementarity determining regions (CDRs), namely CDR1, CDR2, and CDR3. The most important CDR3 is responsible for directly binding to the peptide presented by MHC. CDR1 of the α subunit and the β subunit act on the N-terminus and C-terminus of the polypeptide, respectively. CDR2 is believed to participate in recognizing MHC. The β subunit also has an additional CDR4, which generally does not participate in the recognition of the peptide/MHC complex but is related to the action of superantigens.

As used herein, the major histocompatibility complex (MHC) is a gene family present in the genome of most vertebrates, and is a molecule for antigen presentation and T cell activation. The human MHC glycoprotein is also known as human leukocyte antigen (HLA). MHC molecules include class I and class II MHC molecules. MHC molecules can present degraded fragments of intracellular proteins. For example, after a cell is infected by a virus, the corresponding viral envelope polypeptide fragments can be presented on the cell surface via MHC molecules for recognition by cytotoxic T cells (CD8+ cytotoxic T cells), which then specifically kill the cells infected by the virus.

As used herein, amino acid residues are described using the following abbreviations: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), valine (Val or V). Furthermore, in the present specification, the amino acid sequence of a peptide is described in a conventional manner with the amino terminus (hereinafter referred to as the N-terminus) on the left and the carboxyl terminus (hereinafter referred to as the C-terminus) on the right.

The term "T cell receptor targeting the KRAS G12V mutation (KRAS^{G12V}-reactive T Cell Receptor)" is defined herein as a TCR molecule that is capable of binding to a KRAS G12V mutant polypeptide/MHC complex, thereby inducing T cell cytotoxicity. In particular, the KRAS G12V mutant polypeptide includes, but is not limited to, the amino acid sequence set forth in SEQ ID NO: 16, and the MHC is HLA-A*11:01.

The term "exogenous T cell receptor" (exogenous TCR) is defined herein as a recombinant TCR expressed in a cell by introduction of an exogenous coding sequence. The TCR targeting the KRAS G12V mutation provided herein is an "exogenous T cell receptor" for human T cells, which can be expressed in human T cells that naturally express an endogenous TCR, which is insufficient to induce a response of the cell or a responder cell to TCR ligand binding.

As used herein, TCR-T cell therapy involves introducing an exogenous TCR gene into ordinary T cells, enabling the modified T cells to express a TCR that can effectively recognize tumor cells, thereby directing the T cells to kill the tumor cells. This therapy generally comprises the step of administering to a patient T cells that have been modified to express an exogenous TCR gene. The T cells are usually derived from the patient themselves. Typically, T cells are obtained from the patient, modified ex vivo to express an exogenous TCR gene (such as the TCR gene according to any embodiment herein), and then infused back into the patient.

As used herein, a dual-targeting protein molecule is a class of artificial protein molecules designed based on the BiTE (Bi-specific T-cell engagers) strategy. One end of the protein is a high-affinity T cell receptor (TCR), which can target the KRAS G12V mutation on the surface of tumor cells; the other end is a single-chain antibody (scFv), which is used to recruit and redirect immune cells to the periphery of tumor cells. An exemplary single-chain antibody can be an anti-CD3 single-chain antibody, and an exemplary immune cell can be a T cell. The TCR first recognizes and binds to the polypeptide/MHC on the surface of tumor cells. Then, the anti-CD3 antibody fragment recruits and redirects immune cells to the periphery of tumor cells. In this way, the dual-targeting protein molecule builds a bridge between cancer cells and immune cells, forms an immune synapse, activates immune cells, and releases cytolytic granules, leading to cancer cell death.

As used herein, sequence identity can be determined by methods known in the art, for example, BLASTP can be used to determine the sequence identity of two aligned amino acid sequences. "Conservative substitutions" are known in the art as substitutions in which one or more amino acid residues are replaced by one or more amino acid residues having side chain R groups with similar chemical properties (e.g., charge or hydrophobicity). Generally, conservative amino acid substitutions do not substantially alter the functional properties of a protein. Examples of groups of amino acids having side chains with similar chemical properties include: 1. aliphatic side chains: glycine, alanine, valine, leucine, and isoleucine; 2. aliphatic hydroxyl side chains: serine and threonine; 3. amide-containing side chains: asparagine and glutamine; 4. aromatic side chains: phenylalanine, tyrosine, and tryptophan; 5. basic side chains: lysine, arginine, and histidine; 6. acidic side chains: aspartic acid and glutamic acid; and 7. sulfur-containing side chains: cysteine and methionine. Amino acids can also be classified according to the polarity of their side chain groups as follows: 1. non-polar amino acids (hydrophobic amino acids), including alanine, valine, leucine, isoleucine, proline, phenylalanine, tryptophan, and methionine; 2. polar amino acids (hydrophilic amino acids), including polar uncharged (neutral amino acids) such as glycine, serine, threonine, cysteine, tyrosine, asparagine, glutamine, selenocysteine, and pyrrolysine, and polar positively charged amino acids (basic amino acids), including lysine, arginine, and histidine; 3. polar negatively charged amino acids (acidic amino acids), including aspartic acid and glutamic acid.

As used herein, immune cells refer to cells involved in or related to an immune response, generally including lymphocytes, dendritic cells, monocytes/macrophages, granulocytes, mast cells, and the like. Exemplary immune cells include T cells, natural killer (NK) cells, tumor infiltrating lymphocytes (TIL), and the like, as well as immune cells derived therefrom, such as stem cells, e.g., hematopoietic stem cells (HSC) and induced pluripotent stem cells (iPS), and the like.

### T Cell Receptor (TCR)

The characteristics of the TCR molecule targeting the KRAS G12V mutation of the present invention include that the CDR3 sequence of the α chain variable region thereof comprises CAVNPNTGNQFYF (SEQ ID NO: 1) or a mutant of SEQ ID NO: 1, and/or the CDR3 sequence of the β chain variable region thereof comprises CASSQDYGPQETQYF (SEQ ID NO: 2) or a mutant of SEQ ID NO: 2. Preferably, compared to SEQ ID NO: 1, the mutant of SEQ ID NO: 1 has 1-5 (e.g., 1, 2, or 3) amino acid mutations, or has at least 80%, preferably at least 85%, preferably at least 90%, preferably at least 95%, preferably at least 98% sequence identity, and retains the binding activity of SEQ ID NO: 1 as the CDR3 of the TCR α chain variable region. Preferably, compared to SEQ ID NO: 2, the mutant of SEQ ID NO: 2 has 1-5 (e.g., 1, 2, or 3) amino acid mutations, or has at least 80%, preferably at least 85%, preferably at least 90%, preferably at least 95%, preferably at least 98% sequence identity, and retains the binding activity of SEQ ID NO: 2 as the CDR3 of the TCR β chain variable region. The mutations contained in the mutants of SEQ ID NO: 1 and SEQ ID NO: 2, respectively, can be selected from one or more of insertion, deletion, and substitution. Preferably, the mutation is a conservative mutation, such as a conservative substitution.

In some embodiments, the CDR1 sequence of the α chain variable region of the TCR of the present invention comprises SSSVPPY (SEQ ID NO: 3) or a mutant thereof, and the CDR2 sequence comprises KYTSAAT (SEQ ID NO: 4) or a mutant thereof. In some embodiments, the CDR1 sequence of the β chain variable region of the TCR of the present invention comprises LGHDTM (SEQ ID NO: 5) or a mutant thereof, and the CDR2 sequence comprises SYNNKE (SEQ ID NO: 6) or a mutant thereof. The mutants of SEQ ID NO: 3, 4, 5, and 6, respectively, can have 1, 2, or 3 amino acid mutations compared to themselves, including but not limited to one or more of insertion, deletion, and substitution, and such mutations do not affect the biological function and activity of these CDR sequences in the TCR molecule. Preferred mutations are conservative mutations, such as conservative substitutions.

In some embodiments, the CDR1 sequence of the α chain variable region of the TCR molecule of the present invention is SSSVPPY (SEQ ID NO: 3), the CDR2 sequence is KYTSAAT (SEQ ID NO: 4), and the CDR3 sequence is CAVNPNTGNQFYF (SEQ ID NO: 1); and/or the CDR1 sequence of the β chain variable region is LGHDTM (SEQ ID NO: 5), the CDR2 sequence is SYNNKE (SEQ ID NO: 6), and the CDR3 sequence is CASSQDYGPQETQYF (SEQ ID NO: 2).

The CDR region amino acid sequences of the TCR molecule of the present invention can be grafted into any suitable framework structure to prepare a chimeric TCR. As long as the framework structure is compatible with the CDR regions of the TCR of the present invention, a person skilled in the art can design or synthesize a TCR molecule having corresponding functions based on the CDR regions disclosed in the present invention. Therefore, the TCR molecule of the present invention refers to a TCR molecule that comprises the above-described α and/or β chain CDR region sequences and any suitable framework structure, wherein the CDR region sequences of the present invention are employed.

In some embodiments, the α chain variable region of the TCR molecule of the present invention comprises the amino acid sequence shown in SEQ ID NO: 7, or comprises an amino acid sequence having one or more mutations compared to the amino acid sequence shown in SEQ ID NO: 7, or comprises an amino acid sequence having at least 80%, at least 85%, at least 90%, preferably at least 95%, more preferably at least 98% sequence identity compared to the amino acid sequence shown in SEQ ID NO: 7, or consists of said amino acid sequence. The number of mutated amino acid residues can be, for example, 1-15, such as 1-10 or 1-5 mutations; the mutations can be selected from one or more of insertion, deletion, and substitution. Mutations can occur in any domain of SEQ ID NO: 7, including in the CDR and/or FR regions thereof. In some embodiments, the mutations do not occur in the CDR1, CDR2, and CDR3 sequences comprised in SEQ ID NO: 7. In some embodiments, the mutations occur, for example, in the FR region of SEQ ID NO: 7. Preferably, the mutations are conservative mutations, such as conservative substitutions.

In some embodiments, the β chain variable region of the TCR molecule of the present invention comprises the amino acid sequence shown in SEQ ID NO: 8, or comprises an amino acid sequence having one or more mutations compared to the amino acid sequence shown in SEQ ID NO: 8, or comprises an amino acid sequence having at least 80%, at least 85%, at least 90%, at least 95%, at least 98% sequence identity compared to the amino acid sequence shown in SEQ ID NO: 8, or consists of said amino acid sequence. The number of mutated amino acid residues can be, for example, 1-15, such as 1-10 or 1-5 mutations; the mutations can be selected from one or more of insertion, deletion, and substitution. Mutations can occur in any domain of SEQ ID NO: 8, including in the CDR and/or FR regions thereof. In some embodiments, the mutations do not occur in the CDR1, CDR2, and CDR3 sequences comprised in SEQ ID NO: 8. In some embodiments, the mutations occur, for example, in the FR region of SEQ ID NO: 8. Preferably, the mutations are conservative mutations, such as conservative substitutions.

It should be understood that the mutant of the TCR molecule of the present invention (i.e., the mutant comprising the above-described α chain variable region mutant and/or β chain variable region mutant) still retains the biological activity of the TCR molecule comprising SEQ ID NO: 1 and SEQ ID NO: 2 (particularly the TCR molecule comprising SEQ ID NO: 7 and SEQ ID NO: 8) to specifically bind to the KRAS G12V mutation (particularly the polypeptide shown in SEQ ID NO: 16) in an HLA-A*11:01-dependent manner.

In some embodiments, the TCR molecule of the present invention is a dimer composed of α and β chains, wherein the α chain comprises a variable region and a constant region, the CDR1 sequence of the α chain variable region is SSSVPPY (SEQ ID NO: 3), the CDR2 sequence is KYTSAAT (SEQ ID NO: 4), and the CDR3 sequence is CAVNPNTGNQFYF (SEQ ID NO: 1); and the CDR1 sequence of the β chain variable region is LGHDTM (SEQ ID NO: 5), the CDR2 sequence is SYNNKE (SEQ ID NO: 6), and the CDR3 sequence is CASSQDYGPQETQYF (SEQ ID NO: 2). In some embodiments, the α chain variable region comprises the amino acid sequence shown in SEQ ID NO: 7 or the mutant sequence described above, and the β chain variable region comprises the amino acid sequence shown in SEQ ID NO: 8 or the mutant sequence described above. To further stabilize the formation of the TCR α and β chain dimer, a disulfide bond can be introduced between the α chain and the β chain to form the dimer.

In some embodiments, the constant region of the TCR molecule of the present invention is a human constant region. The amino acid sequence of the human constant region can be obtained from publicly available databases.

Studies have shown that replacing the constant region of a human TCR with a mouse TCR constant region can effectively avoid the issue of an exogenous T cell receptor rearranging with an endogenous TCR in the human body, which would otherwise cause off-target effects or even binding to incorrect targets. Therefore, in some embodiments, the TCR molecule of the present invention comprises a mouse α constant region and a mouse β constant region. An exemplary amino acid sequence of the mouse α constant region is shown in SEQ ID NO: 9, and an exemplary amino acid sequence of the mouse β constant region is shown in SEQ ID NO: 10.

In some embodiments, the TCR of the present invention is provided in the form of a multivalent complex. The multivalent TCR complex of the present invention comprises a polymer formed by combining two, three, four, or more TCR molecules of the present invention.

### Nucleic Acid Molecule

The present invention provides a nucleic acid molecule encoding the α chain variable region, β chain variable region, α chain, β chain, and TCR molecule according to any embodiment described herein.

The nucleotide sequence of the nucleic acid molecule of the present invention can be single-stranded or double-stranded. The nucleic acid molecule can be RNA or DNA, and may contain or not contain introns. An exemplary sequence of the nucleic acid molecule encoding the α chain variable region of the present invention is shown in SEQ ID NO: 11. An exemplary polynucleotide sequence encoding the β chain variable region of the present invention is shown in SEQ ID NO: 12.

In some embodiments, the TCR molecule of the present invention comprises a human variable region and a mouse constant region. In the mouse constant region, the nucleic acid coding sequence of the α constant region can be as shown in SEQ ID NO: 17, and the nucleic acid coding sequence of the β constant region can be as shown in SEQ ID NO: 18.

It should be understood that, due to the degeneracy of the genetic code, different nucleotide sequences can encode the same polypeptide. Therefore, the nucleic acid sequence encoding the α chain variable region, β chain variable region, α chain, β chain, and TCR molecule of the present invention can be identical to the nucleic acid sequence shown herein or can be a degenerate variant. To illustrate with one example of the present invention, a "degenerate variant" refers to a nucleic acid sequence that encodes a protein sequence having SEQ ID NO: 7 or 8 but differs from the sequence of SEQ ID NO: 7 or 8.

To efficiently express the TCR in T cells, the nucleotide sequence of the present invention can be optimized using codon optimization methods. Different cells differ in their utilization of specific codons. Codons in the sequence can be altered based on the cell type to increase expression levels. Codon usage tables for mammalian cells and many other organisms are well known to those skilled in the art.

The full-length sequence or fragment of the nucleic acid molecule of the present invention can generally be obtained using, but not limited to, PCR amplification, recombination, or artificial synthesis methods. At present, it is already possible to obtain the DNA sequence encoding the TCR (or fragment thereof, or derivative thereof) of the present invention entirely by chemical synthesis. This DNA sequence can then be introduced into various existing DNA molecules (e.g., vectors), mRNA, or cells known in the art. The DNA or mRNA can be the coding strand or the non-coding strand.

### Nucleic Acid Construct

The present invention also includes a nucleic acid construct comprising the nucleic acid molecule according to any embodiment described herein.

The nucleic acid construct herein can be an expression cassette, which comprises an operably linked promoter sequence, the nucleic acid molecule according to any embodiment described herein, and a polyA tail. The expression cassette can also contain other regulatory elements operably linked to the above elements, such as an enhancer and the like.

In some embodiments, the nucleic acid construct is a vector. As used herein, vectors include but are not limited to expression vectors and cloning vectors. An expression vector refers to a vector used for expressing the TCR of the present invention in vivo or in vitro, and a cloning vector refers to a vector used in the preparation of the nucleic acid molecule of the present invention. An expression vector generally includes expression regulatory elements. Expression regulatory elements are well known in the art and include, but are not limited to, promoters and enhancers. In some embodiments, the vector comprises the expression cassette.

As used herein, an expression vector can be a related vector based on a viral delivery system, including but not limited to adenovirus vectors, adeno-associated virus (AAV) vectors, herpesvirus vectors, retrovirus vectors, lentivirus vectors, and baculovirus vectors; or a non-viral delivery system vector, including but not limited to transposon-based expression vectors, vectors based on gene editing methods, and the like. Ideally, a suitable vector can transfer the TCR nucleic acid of the present invention into a cell, such as a T cell, so that the cell expresses a TCR specific for a KRAS G12V mutant antigen.

### Dual-Targeting Protein Molecule

In some embodiments, the present invention provides a dual-targeting protein molecule capable of simultaneously binding to a tumor cell and an immune cell (particularly a T cell). The dual-targeting protein molecule comprises the TCR molecule according to any embodiment described herein that can target the KRAS G12V mutation on the surface of a tumor cell, and a single-chain antibody (scFv) for recruiting and redirecting an immune cell to the periphery of the tumor cell. The TCR molecule may comprise an α chain variable region and an α chain constant region, as well as a β chain variable region and a β chain constant region of the TCR molecule. The variable region and the constant region may be directly connected, or may be connected via a flexible peptide chain. Typically, the signal peptide and the transmembrane domain in the α chain variable region and the β chain variable region are deleted.

The single-chain antibody is a monoclonal antibody of interest having the described biological functions. An exemplary single-chain antibody can be an anti-CD3 single-chain antibody.

Typically, in the dual-targeting protein molecule, the α chain and the β chain of the TCR molecule form a heterodimer, and the scFv is connected to the N-terminus of the β chain variable region of the TCR molecule. The scFv and the N-terminus of the β chain variable region may be directly connected, or may be connected via a flexible peptide chain.

As used herein, a flexible peptide chain can be any peptide chain without secondary structure. Suitable flexible peptide chains (linkers) are well known in the art and generally contain G and S. Exemplary flexible linkers include, but are not limited to, sequences containing G and S or consisting of G and S that are 2-30, such as 3-20 or 3-10, amino acid residues in length. An exemplary amino acid sequence of a linker is shown in SEQ ID NO: 19.

In some embodiments, to obtain a stable dual-targeting protein molecule, cysteine mutations can be made at appropriate sites in the α and β chain constant regions to introduce a disulfide bond to stabilize the dimer structure. It should be noted that, whether or not the above-described artificially introduced disulfide bond is present between the constant regions, the TCR molecule of the present invention can be linked via the natural disulfide bond present in the TCR.

### Cell

The present invention also relates to a host cell produced by genetic engineering using the vector or nucleic acid molecule of the present invention. The term "host cell" refers to any type of cell that can contain the nucleic acid molecule or vector of the present invention or express the TCR molecule or dual-targeting protein molecule of the present invention. In some embodiments, the characteristics of the host cell include: comprising the vector of the present invention or having the nucleic acid molecule of the present invention integrated into the chromosome, and/or expressing the TCR molecule and/or dual-targeting protein molecule according to any embodiment described herein.

Host cells suitable for expressing the TCR of the present invention include, but are not limited to, prokaryotic cells and eukaryotic cells, such as Escherichia coli, yeast cells, insect cells, Chinese hamster ovary (CHO) cells, African green monkey kidney cells (Vero cells), COS cells, HEK293 cells, and the like. The host cell is preferably a peripheral blood lymphocyte (PBL) or a peripheral blood mononuclear cell (PBMC). More preferably, the host cell is a primary T cell.

In some embodiments, the present invention particularly relates to an immune cell, especially a T cell, comprising the vector of the present invention or having the nucleic acid molecule of the present invention integrated into the chromosome, and/or expressing the TCR molecule according to any embodiment described herein. The T cell can be any type of T cell and can be at any stage of development, including but not limited to: CD4+/CD8+ double-positive T cells, CD4+ helper T cells (e.g., Th1 and Th2 cells), CD4+ T cells, CD8+ T cells (e.g., cytotoxic T cells), memory T cells (e.g., central memory T cells and effector memory T cells), naive T cells, and the like. More preferably, the T cell can be derived from CD8+ T cells isolated from a patient.

In some embodiments, the cell of the present invention can also be other types of immune cells, such as natural killer (NK) cells, tumor infiltrating lymphocytes (TIL), and derived immune cells. Gene transfer into NK cells will not result in expression of TCR on the cell surface because NK cells do not express the CD3 molecule. However, when NK cells are induced to differentiate or are artificially engineered, expression of the CD3 molecule will initiate expression of the TCR molecule in the NK cells.

### Pharmaceutical Composition and Conjugate

The present invention also provides a pharmaceutical composition comprising a T cell and a pharmaceutically acceptable carrier, wherein the T cell comprises a vector expressing the TCR molecule according to any embodiment described herein or has a nucleic acid molecule encoding the TCR molecule according to any embodiment described herein integrated into its chromosome, and/or expresses the TCR molecule according to any embodiment described herein.

In some embodiments, the pharmaceutical composition of the present invention comprises the TCR molecule or the dual-targeting protein molecule according to any embodiment described herein and a pharmaceutically acceptable carrier.

As used herein, the pharmaceutically acceptable carrier can be selected according to the specific active ingredient. For example, the pharmaceutically acceptable carrier in the pharmaceutical composition comprising the T cell can be various suitable carriers well known in the art and suitable for cell therapy. The pharmaceutically acceptable carrier in the pharmaceutical composition comprising the TCR molecule or the dual-targeting protein molecule of the present invention can be a pharmaceutically acceptable carrier suitable for protein delivery.

As used herein, the pharmaceutical composition can be administered by any suitable route, such as parenteral, enteral, inhalation, or intranasal routes. The pharmaceutical composition of the present invention can be prepared by methods well known in the art, for example, by mixing the active ingredient with a carrier or excipient under sterile conditions.

The effective amount of the active ingredient, such as the T cell, the TCR molecule, or the dual-targeting protein molecule, in the pharmaceutical composition of the present invention depends on the disease or disorder to be treated, the age and condition of the individual to be treated, and the like, and can be readily determined by a person skilled in the art according to the actual situation. Generally, a suitable dosage range for the soluble TCR of the present invention can be between 25 ng/kg and 50 µg/kg.

The pharmaceutical composition of the present invention can be used for various therapeutic purposes as described below.

In some embodiments, the present invention provides a conjugate, which comprises the TCR molecule described herein and a therapeutic agent or tracer covalently or otherwise bound to the TCR molecule, for use in the treatment or diagnosis of a disease, particularly a tumor. The bound or conjugated therapeutic agents include, but are not limited to: radionuclides, chemotherapeutic agents, antibody Fc or scFv fragments, nanoparticles, and the like. The tracer for diagnostic purposes includes, but is not limited to: a fluorescent or luminescent label, a radioactive label, a magnetic material for MRI (magnetic resonance imaging), a contrast agent for CT (computed tomography), and an enzyme that produces a detectable product.

### Use and Treatment Method

The present invention also provides use of the TCR molecule, the dual-targeting protein molecule, and the cell (particularly the T cell) according to any embodiment described herein in the manufacture of a medicament for treating or preventing a disease related to a KRAS G12V mutant antigen in a patient, as well as the TCR molecule, the dual-targeting protein molecule, and the cell (particularly the T cell) according to any embodiment described herein for use in treating or preventing a disease related to a KRAS G12V mutant antigen.

The present invention also relates to a method for treating and/or preventing a disease related to a KRAS G12V mutant antigen in a patient, comprising the step of adoptively transferring to the patient a T cell comprising the vector of the present invention or having the nucleic acid molecule of the present invention integrated into its chromosome, and/or expressing the TCR molecule according to any embodiment described herein, or comprising the step of administering to the patient the dual-targeting protein molecule according to any embodiment described herein or a pharmaceutical composition comprising the dual-targeting protein molecule.

As used herein, the disease related to a KRAS G12V mutant antigen is a tumor or cancer, and may include any one of the following: acute lymphocytic cancer, acute myeloid leukemia, alveolar rhabdomyosarcoma, bone cancer, brain cancer, breast cancer, anal cancer, anal canal cancer or anorectal cancer, eye cancer, intrahepatic cholangiocarcinoma, joint cancer, cervical cancer, gallbladder cancer or pleural cancer, nasal cancer, nasal cavity cancer or middle ear cancer, oral cancer, vaginal cancer, vulvar cancer, chronic lymphocytic leukemia, chronic myeloid cancer, colon cancer, colorectal cancer, endometrial cancer, esophageal cancer, cervical cancer, gastrointestinal carcinoid tumor, glioma, Hodgkin's lymphoma, hypopharyngeal cancer, kidney cancer, laryngeal cancer, liver cancer, lung cancer, malignant mesothelioma, melanoma, multiple myeloma, nasopharyngeal cancer, non-Hodgkin's lymphoma, oropharyngeal cancer, ovarian cancer, penile cancer, pancreatic cancer, peritoneal cancer, omental cancer and mesenteric cancer, pharyngeal cancer, prostate cancer, rectal cancer, renal cancer, skin cancer, small intestine cancer, soft tissue cancer, stomach cancer, testicular cancer, thyroid cancer, uterine cancer, ureteral cancer, and bladder cancer. Preferred cancers are pancreatic cancer, colorectal cancer, lung cancer, endometrial cancer, ovarian cancer, or prostate cancer. In some embodiments, the disease is pancreatic cancer.

Preferably, the tumor cell or cancer cell of the patient carries the KRAS G12V mutant antigen and HLA-A*11:01. Preferably, the KRAS G12V mutant antigen carried by the tumor cell or cancer cell of the patient includes, but is not limited to, the amino acid sequence as shown in SEQ ID NO: 16.

Treatment can be performed by isolating T cells from a patient or volunteer suffering from a disease related to a KRAS G12V mutant antigen, genetically modifying the T cells ex vivo so that they comprise the vector according to any embodiment described herein or have integrated into their genome a nucleic acid molecule capable of expressing the TCR molecule according to any embodiment described herein, thereby expressing the TCR molecule according to any embodiment described herein, and subsequently infusing these genetically engineered cells back into the patient.

In some embodiments, the T cell is derived from the patient himself/herself. Therefore, in these embodiments, the treatment method of the present invention further comprises: (1) isolating the patient's T cell, and (2) genetically modifying the T cell ex vivo so that it comprises the vector according to any embodiment described herein or has integrated into its genome a nucleic acid molecule capable of expressing the TCR molecule according to any embodiment described herein, thereby expressing the TCR molecule according to any embodiment described herein.

The route, timing, dosage, and the like of administration can be determined by a physician based on factors such as the age, weight, general health status, severity of the cancer being treated of each individual patient.

The TCR, polypeptide, protein, nucleic acid, recombinant expression vector, and host cell (including populations thereof) of the present invention can be formulated into a pharmaceutical composition in combination with another pharmaceutically active agent or drug. The another pharmaceutically active agent or drug can be a chemotherapeutic agent, such as asparaginase, busulfan, carboplatin, cisplatin, daunorubicin, doxorubicin, fluorouracil, gemcitabine, hydroxyurea, methotrexate, paclitaxel, rituximab, vinblastine, vincristine, and the like. It can also be a monoclonal antibody-based therapeutic drug, such as an immune checkpoint-targeting antibody drug (CTLA-4, PD1, PD-L1, TIGIT, LAG3, TIM3, etc.), or an immune regulatory element-targeting antibody drug (4-1BB, OX40, GITR, CD40, CD28, ICOS, CD47, etc.). In addition, it also includes other types of tumor therapeutic agents, such as oncolytic viruses and vaccines (including but not limited to mRNA, DNA, protein, protein subunit, cell component, or cell, etc.).

The following specific examples further illustrate the present invention. It should be understood that these examples are for illustrative purposes only and are not intended to limit the scope of the present invention. In the following examples, experimental methods without specific conditions indicated are generally performed under conventional conditions, such as those described in Sambrook and Russell et al., Molecular Cloning: A Laboratory Manual (Third Edition) (2001), CSHL Press, or according to the conditions recommended by the manufacturer. Unless otherwise indicated, percentages and parts are by weight. Unless otherwise indicated, percentages and parts are by weight. The experimental materials and reagents used in the following examples, unless otherwise specified, can be obtained from commercial sources.

### Example 1: Determination of TCR Gene Sequence Targeting KRAS G12V Mutation

Functional cell populations were sorted from peripheral blood mononuclear cell (PBMC) samples of a tumor patient carrying the KRAS G12V mutation (derived from the Department of General Surgery, Jinling Hospital Affiliated to the Medical School of Nanjing University). TCR sequences were obtained by single-cell sequencing. After functional validation, the TCR designated 02-1 was shown to specifically bind to the VVGAVGVGK/HLA-A*11:01 complex or the VVVGAVGVGK/HLA-A*11:01 complex. The amino acid sequence and coding sequence of its α variable region are shown in SEQ ID NO: 7 and SEQ ID NO: 11, respectively, and the amino acid sequence and coding sequence of its β variable region are shown in SEQ ID NO: 8 and SEQ ID NO: 12, respectively.
SEQ ID NO:7
   MLLLLUPVLEVIFTLGGTRAQSVTQLGSHVSVSEGALVLLRCNY **SSSVPPY**LFWYVQ YPNQGLQLLL**KYTSAAT**LVKGINGFEAEFKKSETSFHLTKPSAHMSDAAEYF**CAVNPNTG NQFYF**GTGTSLTVIP (The sequences of CDR1, CDR2, and CDR3 were indicated in bold and underlined, respectively.)
SEQ ID NO:8
   MGCRLLCCVVFCLLQAGPLDTAVSQTPKYLVTQMGNDKSIKCEQN**LGHDTM**YWYK QDSKKFLKIMF**SYNNKE**LIINETVPNRFSPKSPDKAHLNLHINSLELGDSAVYF**CASSQDY GPQETQYF**GPGTRLLVL (The sequences of CDR1, CDR2, and CDR3 were indicated in bold and underlined, respectively.)

### Example 2: Construction of Vector with High Expression of TCR Molecule

### 1. Vector Information

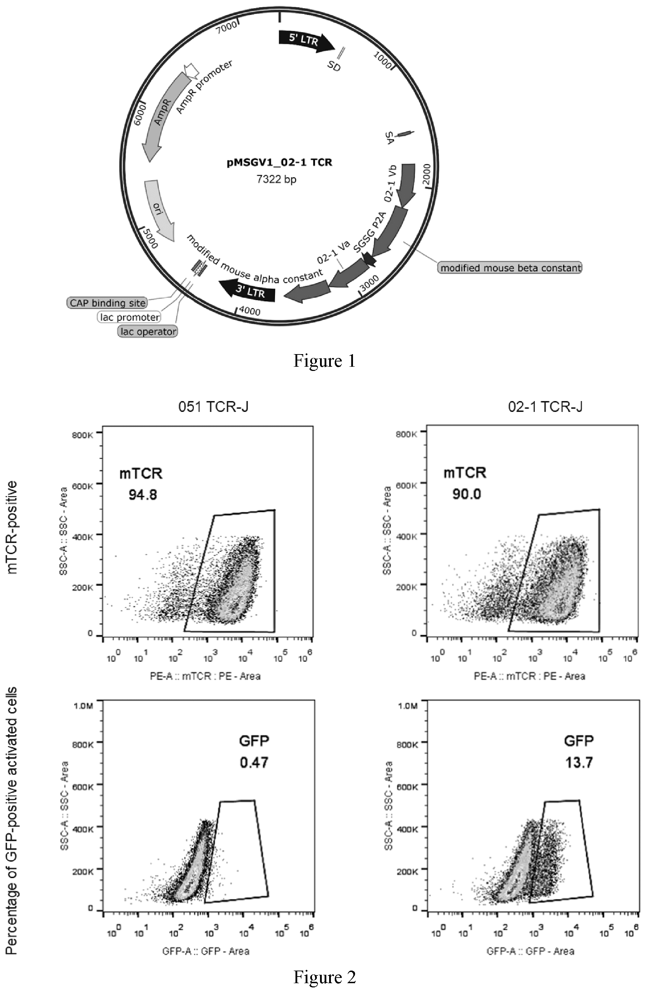

The pMSGV1 vector was used to overexpress the TCR molecule in T cells. Codon optimization for human expression was used for the TCR nucleic acid sequence. The coding sequence of the α chain variable region was shown in SEQ ID NO: 11; the coding sequence of the β chain variable region was shown in SEQ ID NO: 12. Furthermore, expression of the TCR in T cells was achieved using a human-mouse chimeric approach. The amino acid sequence of the mouse α constant region was shown in SEQ ID NO: 9, and the amino acid sequence of the mouse β constant region was shown in SEQ ID NO: 10. The TCR α chain and β chain were linked in tandem using an SGSG-P2A sequence (amino acid sequence SEQ ID NO: 20, nucleic acid sequence SEQ ID NO: 21). The complete vector structure was shown in Figure 1. After the vector construction was completed, sequencing was performed for identification, and the sequencing primers were shown in SEQ ID NO: 22, SEQ ID NO: 23, SEQ ID NO: 24, and SEQ ID NO: 25.

### 2. Plasmid Extraction

After sequencing was confirmed correct, NucleoBond Xtra Maxi (MACHEREY-NAGEL) was used to extract and purify the plasmid. The purified plasmid was subjected to UV spectrophotometry to measure absorbance at 259 nm to calculate the plasmid concentration, and was stored at -20°C for subsequent experiments.

### Example 3: Retrovirus Packaging

HEK 293 cells were transiently transfected with retrovirus packaging plasmids to prepare retroviral vectors for infecting target cells to generate TCR-T cells or reporter cells expressing the 02-1 TCR. The specific procedure was as follows:
1. Day 1: HEK 293 cells were digested and plated at a density of 0.6×10⁶ cells/mL. To a T25 flask, 5 mL of D10 medium (DMEM + 10% FBS) was added, and the cells were mixed thoroughly and cultured overnight at 37°C.
2. Day 2: When the confluence of HEK 293 cells reached about 90%, transfection was performed. A plasmid complex was prepared. The amounts of the various plasmids were as follows: pMSGV1-02-1 TCR 3 µg, Gag-pol 1.9 µg, 10A1 0.75 µg. 300 µL of DMEM was added. 20 µL of EZ Trans cell transfection reagent (Shanghai Liji Biotechnology) was added to 300 µL of DMEM. The PEI solution was added to the plasmid complex and vortexed for 20 seconds. The mixture was gently added along the wall to the HEK 293 culture flask, and the cells were cultured at 37°C for 16 hours. The medium was removed, and pre-warmed fresh medium was added.
3. Day 4: After 48 hours of transfection, the supernatant was collected, filtered through a 0.45 µm filter, aliquoted, and stored at -80°C.

The resulting retrovirus was designated as the 02-1 TCR retrovirus.

### Example 4: Preparation of TCR-J Reporter Cells and In Vitro Antigenic Peptide Activation Assay

A GFP reporter gene was knocked into the Nur77 gene locus of human T lymphoblastic leukemia Jurkat cells, and CD8 and HLA-A*11:01 genes were stably transfected to construct a T cell activation reporter cell line (S1-1-1-CD8-A11). The S1-1-1-CD8-A11 reporter cells were infected with the 02-1 TCR retrovirus prepared in Example 3 to generate 02-1 TCR-J cells, which were then activated in vitro with a KRAS mutant antigenic peptide to test TCR function. The TCR 051 published in CN117264043 was used as a control group. The specific procedure was as follows.

### Preparation of 02-1 TCR-J reporter cells:

1. S1-1-1-CD8-A11 reporter cells were collected and resuspended in R10 medium (RPMI1640 + 10% FBS) to a cell density of 5×10⁵/mL. The cells were seeded into a 24-well culture plate at 1 mL per well.
2. 300 µL of 02-1 TCR retrovirus and 0.65 µL of polybrene (Santa Cruz) were added to each well. The plate was centrifuged at 2,500 rpm for 90 minutes at 32°C.
3. After centrifugation, 800 µL of supernatant was discarded, and 800 µL of fresh R10 medium was added. The culture plate was placed in a 37°C, 5% CO₂ incubator for culture.

### Antigenic peptide activation functional assay:

1. Two days after infection of S1-1-1-CD8-A11 reporter cells with the 02-1 TCR retrovirus, the cells were counted, an appropriate amount was taken and centrifuged, and the cells were resuspended in R10 medium. The cell concentration was adjusted to 2×10⁶/mL, and 100 µL/well was added to a 96-well plate.
2. The KRAS G12V mutant 9-mer peptide (SEQ ID NO: 15) was added to a final concentration of 1 µg/mL, mixed thoroughly, and incubated at 37°C for about 4 hours.
3. The cells were transferred to flow cytometry tubes, washed with PBS, and the supernatant was discarded.
4. 100 µL of zombie green (BioLegend, 1:500 dilution) dye was added, and the cells were incubated at room temperature for 10 minutes in the dark, washed with PBS, and the supernatant was discarded.
5. 100 µL of mTCR antibody (1:300 dilution) was added, and the mixture was kept at 4°C for 30 minutes in the dark. PBS was added for washing, and the cells were resuspended. mTCR and GFP were detected by flow cytometry.

The experimental results were shown in Figure 2. The results indicated that the 02-1 TCR could be successfully constructed and expressed on the S1-1-1-CD8-A11 reporter cells. In the presence of the KRAS G12V mutant 9-mer peptide, the 02-1 TCR recognized the KRAS G12V mutant 9-mer peptide and activated the reporter cells.

### Example 5: Preparation of 02-1 TCR-T Cells

The 02-1 TCR retrovirus prepared in Example 3 was used to infect T cells derived from human peripheral blood mononuclear cells (PBMCs) to prepare 02-1 TCR-T cells. The specific procedure was as follows.
1. PBMC cells obtained by Ficoll separation solution (Tianjin Haoyang) were resuspended in X-VIVO (LONZA) medium containing 5% AB serum to adjust the cell density to 2×10⁶/mL.
2. The cells were seeded into culture plates at 1 mL/well, anti-human CD3 antibody (Takara) at 50 ng/mL was added, and then interleukin-2 (Beijing Shuanglu) at 300 IU/mL was added. After stimulation culture for 48 hours, the cells were infected with the virus.
3. On the day following T cell activation culture, Retronectin (Takara) diluted with PBS to a final concentration of 15 µg/mL was used to coat non-tissue treated culture plates, with 300 µL per well for a 24-well plate, and left overnight at 4°C for later use.
4. Two days after T cell activation culture, the coated 24-well plate was taken out, the coating solution was aspirated and discarded, and the virus solution was added to the wells at 1 mL per well. The plate was centrifuged at 32°C, 2000 g for 2 hours.
5. The supernatant was discarded, and 5×10⁵ activated T cells were added to each well of the 24-well plate in a volume of 1 mL. The culture medium was T cell medium supplemented with IL-2 at 300 IU/mL. The plate was centrifuged at 32°C, 1000 g for 10 minutes.
6. After centrifugation, the culture plate was placed in a 37°C, 5% CO₂ incubator for culture.
7. After cell infection, T cell culture medium containing IL-2 at 100 IU/mL was added in due course to expand the cells.

### Example 6: Binding of 02-1 TCR to Peptide-MHC Complexes

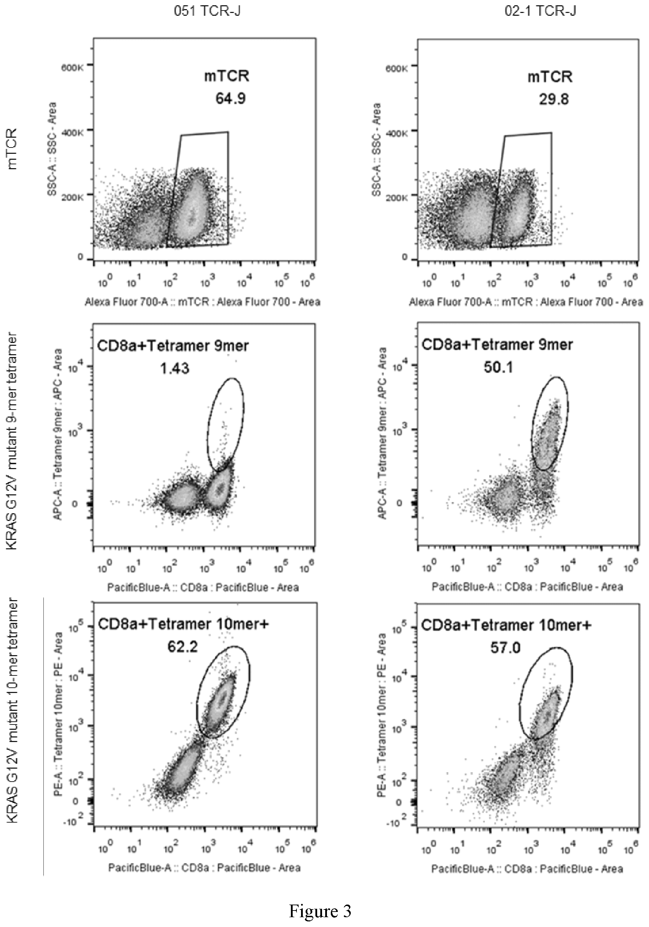

TCR specifically recognizes antigenic peptides presented by MHC complexes. The prepared 02-1 TCR-T cells can be stained with fluorescently labeled peptide-MHC tetramers to detect the binding of the 02-1 TCR to the peptide-MHC complexes. The specific procedure was as follows.
1. The prepared 02-1 TCR-T cells and 051 TCR-T cells (control group) were taken, washed once with PBS, and the supernatant was discarded.
2. 100 µL of mTCR antibody, CD8 antibody (1:300 dilution), and KRAS G12V mutant 9-mer tetramer or KRAS G12V mutant 10-mer tetramer (1:200 dilution) were added, mixed thoroughly, and incubated at 4°C for 30 minutes in the dark.
3. The cells were washed with PBS, resuspended, and detected by flow cytometry. The results were shown in Figure 3.

The results indicated that the 02-1 TCR could bind to the KRAS G12V mutant 9-mer MHC complex and also bind to the KRAS G12V mutant 10-mer MHC complex.

### Example 7: Activation of 02-1 TCR-T Cells by KRAS Mutant Antigenic Peptides

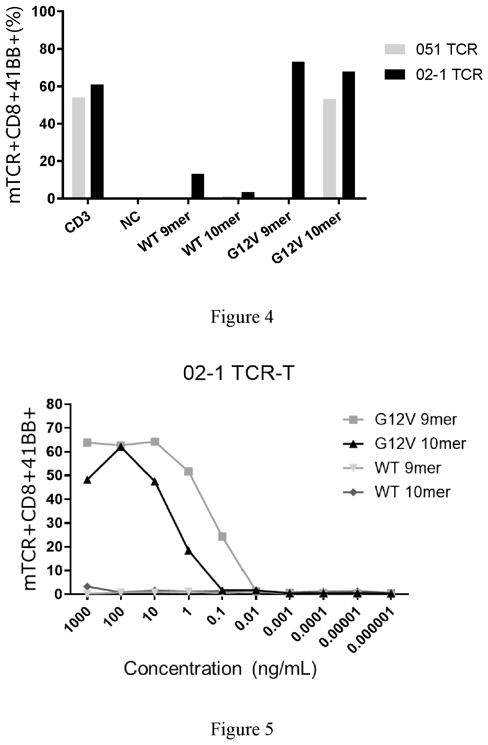

After the TCR on the T cell membrane specifically recognizes an antigenic peptide presented by an MHC complex, the T cell is activated. The activation level of T cells can be reflected by detecting the expression level of CD137 (4-1BB) on the T cell surface. By comparing different antigenic peptides or different concentrations of antigenic peptides, the specificity and sensitivity of TCR recognition can be compared. The specific procedure was as follows.
1. The 02-1 TCR-T cells prepared in Example 6 and 051 TCR-T cells (control group) published in CN117264043 were taken, washed once with PBS, and the supernatant was discarded.
2. The cells were resuspended in X-VIVO15 medium containing 2% AB serum, and the cell concentration was adjusted to 2×10⁶/mL, and 100 µL/well was added to a 96-well plate.
3. Four peptides, KRAS wild-type 9-mer peptide (SEQ ID NO: 13), KRAS wild-type 10-mer peptide (SEQ ID NO: 14), KRAS G12V mutant 9-mer peptide (SEQ ID NO: 15), and KRAS G12V mutant 10-mer peptide (SEQ ID NO: 16), were resuspended in X-VIVO15 medium containing 2% AB serum at a final concentration of 10 ng/mL, and 100 µL/well was added to the 96-well plate containing the T cells. Anti-CD3 antibody (OKT3) was used as a positive control, and a blank solvent containing no antigenic peptide was used as a negative control.
4. After thorough mixing, the plate was placed in a 5% CO₂ incubator and incubated at 37°C for about 16 hours.
5. mTCR+CD8+41BB+ cells were detected by flow cytometry.
   The results were shown in Figure 4. The results indicated that both the KRAS G12V mutant 9-mer and 10-mer peptides could activate the 02-1 TCR-T cells, while the KRAS wild-type 9-mer and 10-mer peptides could not. The recognition of the KRAS G12V mutation by the 02-1 TCR was specific.
6. Four peptides, KRAS wild-type 9-mer peptide (SEQ ID NO: 13), KRAS wild-type 10-mer peptide (SEQ ID NO: 14), KRAS G12V mutant 9-mer peptide (SEQ ID NO: 15), and KRAS G12V mutant 10-mer peptide (SEQ ID NO: 16), were diluted in 10-fold gradients, resuspended in X-VIVO15 medium containing 2% AB serum, and 100 µL/well was added to the 96-well plate containing the T cells, with final concentrations ranging from 10³ ng/mL to 10⁻⁶ ng/mL.
7. After thorough mixing, the plate was placed in a 5% CO₂ incubator and incubated at 37°C for about 16 hours.
8. mTCR+CD8+41BB+ cells were detected by flow cytometry.

The results were shown in Figure 5. The results indicated that both the KRAS G12V mutant 9-mer and 10-mer peptides could activate the 02-1 TCR-T cells, while the KRAS wild-type 9-mer and 10-mer peptides could not. The EC₅₀ for activation of the 02-1 TCR-T by the KRAS G12V mutant 9-mer peptide was 1.85 ng/mL; the EC₅₀ for activation of the 02-1 TCR-T by the KRAS G12V mutant 10-mer peptide was 0.18 ng/mL.

### Example 8: Activation Effect of KRAS Mutant Tumor Cells on 02-1 TCR-T Cells

In tumor cells carrying the KRAS G12V mutation, the KRAS G12V mutant antigenic peptide is presented on the cell surface by MHC molecules and activates TCR-T cells upon contact with the TCR-T cells. Various tumor cells carrying the KRAS G12V mutation were selected and co-incubated with TCR-T cells to detect the activation effect of the 02-1 TCR-T cells. The KRAS G12V mutation was stably expressed in the pancreatic cancer cell line PANC-1 using a retroviral vector to construct PANC-1-LUC-GFP-TMG cells. HLA-A*11:01 was stably expressed in tumor cells naturally carrying the KRAS G12V mutation, SW620, CORL23, and CFPAC1, using retroviral vectors to construct SW620-LUC-GFP-A11, CORL23-LUC-GFP-A11, and CFPAC1-LUC-GFP-A11, respectively, for use in activation experiments of 02-1 TCR-T cells. The specific procedure was as follows.
1. The prepared 02-1 TCR-T cells were taken, counted, an appropriate amount was taken and centrifuged, and the cells were resuspended in X-VIVO medium containing 2% AB serum. The cell concentration was adjusted to 2×10⁶/mL, and 100 µL/well was added to a 96-well plate.
2. Target cells were taken, counted, and the concentration was adjusted to 2×10⁵ to 2×10⁶/mL. The cells were suspended in X-VIVO medium containing 2% AB serum, and 100 µL/well was added to the T cells. CD3 antibody was used as a positive control, and blank medium was used as a negative control.
3. After thorough mixing, the plate was placed in a 5% CO₂ incubator and incubated at 37°C for about 16 hours.
4. mTCR+CD8+41BB+ cells were detected by flow cytometry.

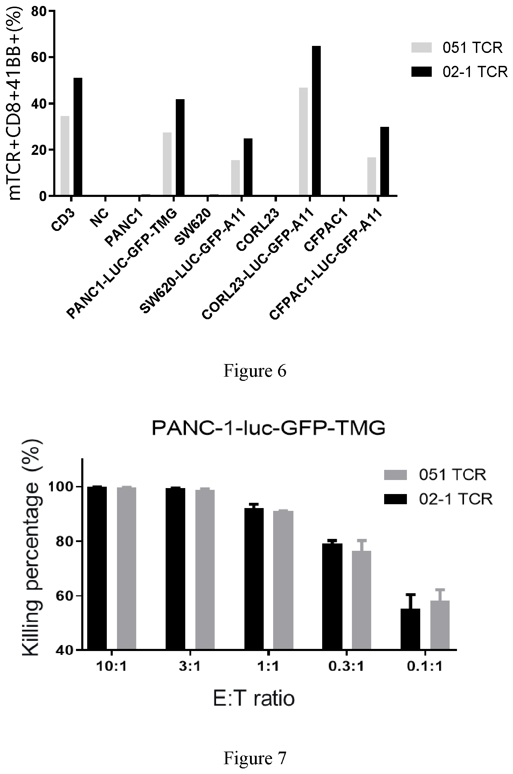

The results were shown in Figure 6. The results indicated that the tumor cells carrying the KRAS G12V mutation, PANC-1-LUC-GFP-TMG, SW620-LUC-GFP-A11, CORL23-LUC-GFP-A11, and CFPAC1-LUC-GFP-A11, could all activate the 02-1 TCR-T cells. In contrast, PANC-1 tumor cells not carrying the KRAS G12V mutation could not activate the 02-1 TCR-T cells. Tumor cells SW620, CORL23, and CFPAC1 carrying the KRAS G12V mutation but whose HLA typing was not HLA-A*11:01 also could not activate the 02-1 TCR-T cells. The activation of the 02-1 TCR-T was specific for both the KRAS G12V mutation and HLA-A*11:01.

### Example 9: Cytotoxic Effect of 02-1 TCR-T Cells on KRAS Mutant Tumor Cells

After TCR-T cells recognize an antigenic peptide epitope presented by MHC on the surface of tumor cells, they are activated and can release effector cytokines such as IFN-γ, as well as cytotoxic molecules such as perforin and granzyme B, causing tumor cell death. The specific procedure was as follows.
1. The prepared 02-1 TCR-T cells were taken, counted, an appropriate amount was taken and centrifuged, and the cells were resuspended in X-VIVO medium containing 2% AB serum. The cell concentration was adjusted to 2×10⁶/mL, and 100 µL/well was added to a 96-well plate.
2. Target cells were taken, counted, and the concentration was adjusted according to different effector-to-target (E:T) ratios. The cells were suspended in X-VIVO medium containing 2% AB serum, and 100 µL/well was added to the T cells. 051 TCR-T cells were used as a control group.
3. After thorough mixing, the plate was placed in a 5% CO₂ incubator and incubated at 37°C for about 16 hours.
4. Luciferase detection reagent was added, and the tumor cell killing percentage was calculated.

The results were shown in Figure 7. The results indicated that the tumor cells carrying the KRAS G12V mutation, PANC-1-LUC-GFP-TMG, could be killed by the 02-1 TCR-T cells at various effector-to-target ratios.

## Claims

1. A T cell receptor (TCR) molecule, wherein the TCR molecule specifically targets the KRAS G12V mutation, the CDR3 sequence of the α chain variable region thereof comprises CAVNPNTGNQFYF (SEQ ID NO: 1) or a mutant of SEQ ID NO: 1, and/or the CDR3 sequence of the β chain variable region thereof comprises CASSQDYGPQETQYF (SEQ ID NO: 2) or a mutant of SEQ ID NO: 2; wherein, compared to SEQ ID NO: 1, the mutant of SEQ ID NO: 1 has 1-5 amino acid mutations or has at least 80% sequence identity, and retains the binding activity of SEQ ID NO: 1 as the CDR3 of the TCR α chain variable region; and compared to SEQ ID NO: 2, the mutant of SEQ ID NO: 2 has 1-5 amino acid mutations or has at least 80% sequence identity, and retains the binding activity of SEQ ID NO: 2 as the CDR3 of the TCR β chain variable region.

2. The TCR molecule according to claim 1, wherein in the TCR molecule:
the CDR1 sequence of the α chain variable region comprises SSSVPPY (SEQ ID NO: 3) or a mutant thereof, and the CDR2 sequence of the α chain variable region comprises KYTSAAT (SEQ ID NO: 4) or a mutant thereof; and/or
the CDR1 sequence of the β chain variable region comprises LGHDTM (SEQ ID NO: 5) or a mutant thereof, and the CDR2 sequence of the β chain variable region comprises SYNNKE (SEQ ID NO: 6) or a mutant thereof.

3. The TCR molecule according to claim 1, wherein the α chain variable region of the TCR molecule comprises the amino acid sequence shown in SEQ ID NO: 7, or comprises or consists of an amino acid sequence having one or more mutations compared to the amino acid sequence shown in SEQ ID NO: 7; and/or the β chain variable region of the TCR molecule comprises the amino acid sequence shown in SEQ ID NO: 8, or comprises or consists of an amino acid sequence having one or more mutations compared to the amino acid sequence shown in SEQ ID NO: 8;
wherein the TCR molecule comprising the mutation(s) retains the biological activity of the TCR molecule comprising SEQ ID NO: 7 and SEQ ID NO: 8 to specifically target a KRAS G12V mutant polypeptide.

4. The TCR molecule according to any one of claims 1-3, wherein the TCR molecule comprises a mouse constant region.

5. The TCR molecule according to claim 4, wherein the amino acid sequence of the mouse α constant region is shown in SEQ ID NO: 9, and the amino acid sequence of the mouse β constant region is shown in SEQ ID NO: 10.

6. A multivalent TCR complex, wherein the multivalent TCR complex comprises two or more TCR molecules according to any one of claims 1-5.

7. A dual-targeting protein molecule capable of simultaneously binding to a tumor cell and an immune cell, wherein the dual-targeting protein molecule comprises the TCR molecule targeting the KRAS G12V mutation on the surface of a tumor cell according to any one of claims 1-5 and a single-chain antibody (scFv) for recruiting and redirecting an immune cell to the periphery of the tumor cell, wherein the signal peptide and the transmembrane domain in the α chain variable region and the β chain variable region of the TCR molecule are deleted.

8. The dual-targeting protein molecule according to claim 7, wherein the single-chain antibody is an anti-CD3 single-chain antibody.

9. A nucleic acid molecule, wherein the nucleic acid molecule comprises a nucleic acid sequence encoding the TCR molecule according to any one of claims 1-5 or the dual-targeting protein molecule according to claim 7 or 8, or a complementary sequence thereof.

10. The nucleic acid molecule according to claim 9, wherein the nucleic acid sequence of the nucleic acid molecule is selected from SEQ ID NO: 11 and SEQ ID NO: 12.

11. A nucleic acid construct, wherein the nucleic acid construct comprises the nucleic acid molecule according to claim 9 or 10;
preferably, the nucleic acid construct is a vector, preferably an expression vector; preferably, the vector is a viral vector or a non-viral vector; more preferably, the vector is a retroviral vector.

12. An isolated cell, wherein the cell:
(1) comprises the nucleic acid construct according to claim 11 or has the nucleic acid molecule according to claim 9 or 10 integrated into its chromosome, and/or
(2) expresses the TCR molecule according to any one of claims 1-5 or the dual-targeting protein molecule according to claim 7 or 8;
preferably, the cell is an immune effector cell, preferably a T cell, an NK cell, or a TIL cell.

13. A pharmaceutical composition, wherein the pharmaceutical composition comprises a pharmaceutically acceptable carrier and the TCR molecule according to any one of claims 1-5, the TCR complex according to claim 6, the dual-targeting protein molecule according to claim 7 or 8, the nucleic acid molecule according to claim 9 or 10, the recombinant expression vector according to claim 11, or the cell according to claim 12.

14. Use of the TCR molecule according to any one of claims 1-5, the TCR complex according to claim 6, the dual-targeting protein molecule according to claim 7 or 8, the nucleic acid molecule according to claim 9 or 10, the recombinant expression vector according to claim 11, or the cell according to claim 12 in the manufacture of a medicament for treating or preventing a disease related to a KRAS G12V mutant antigen in a patient.

15. A method for treating or preventing a disease related to a KRAS G12V mutant antigen in a patient, the method:
comprises a step of adoptively transferring to the patient a T cell comprising the expression vector according to claim 11 or having the nucleic acid molecule according to claim 9 or 10 integrated into its chromosome, and/or expressing the TCR molecule according to any one of claims 1-5, or
comprises a step of administering to the patient the dual-targeting protein molecule according to claim 7 or 8 or a pharmaceutical composition comprising the dual-targeting protein molecule.
